# EUROPEAN PATENT APPLICATION

(11) **EP 4 556 890 A1**
(43) Date of publication of application: **21.05.2025**
(21) Application number: 23209753.5
(22) Date of filing: 14.11.2023
(51) Int. Cl.: G01N 21/64, G01N 33/50, G01N 33/533

(54) **LABEL, MARKER AND REAGENT KIT FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

A label (102) for analysing a biological sample is provided. The label (102) comprises a backbone (106) with at least one aptamer (108), and at least one labelling moiety (110) bound by the aptamer (108). The aptamer (108) is configured to specifically bind to at least one labelling moiety (110) by its complex structure. This enables a compact label (102) that is efficiently generated and assembled. In further aspects, a marker (104) and a reagent kit comprising the label (102) is provided.

## Description

### Technical field

The invention relates to a label for analysing a biological sample comprising at least one aptamer. In further aspects, a marker and a reagent kit for analysing a biological sample comprising the label are provided.

### Background

Markers for fluorescent microscopy generally comprise affinity reagents such as antibodies and labels attached to the affinity reagents in order to enable specifically attaching the labels to a target analyte in a biological sample. This allows the identification and/or localisation of the target analyte in the biological sample. The ubiquitous use of these markers and labels, for example in cyclical staining methods, results in a drive to improve the efficiency of producing these markers and labels.

A particular issue with labels and markers known in the art is that each part of the label or marker is often generated individually. In order to assemble the label or marker, the individual parts need to be assembled, often using specific chemistries and linkers, resulting in a complex production. Specifically, the coupling of labelling moieties like fluorescent dyes is a process that is generally associated with losses due to limited efficiency of coupling reactions and subsequent purification of coupling products.

The use of linkers also increases the overall size of labels and markers. This may be particularly detrimental when using high resolution microscopy.

### Summary

It is an object to provide a compact label and a marker for analysing a biological sample that can be efficiently generated.

The aforementioned object is achieved by the subject-matter of the independent claims. Advantageous embodiments are defined in the dependent claims and the following description.

A label for analysing a biological sample is provided. The label comprises a backbone with at least one aptamer, and at least one labelling moiety bound by the aptamer. The aptamer is configured to specifically bind to at least one labelling moiety by its complex structure. This enables a compact label that is efficiently generated and assembled.

For example, the complex structure of the aptamer of the backbone may be a secondary, tertiary or quaternary structure, in particular, of a nucleic acid of the aptamer. Thus, the aptamer has a secondary, tertiary or quaternary structure, in particular. In particular, the aptamer is configured to bind specifically to the labelling moiety due to the complex structure of the aptamer and based on intermolecular forces between the aptamer and the labelling moiety. Specifically, individual, discontinuous nucleotides of the nucleic acid of the aptamer bind to the labelling moiety, rather than continuous nucleotides of a sequence of the nucleic acid hybridising to the labelling moiety, in particular a complementary sequence of the labelling moiety. This enables binding of the labelling moiety with high specificity.

Preferably, the backbone comprises, in particular, consists essentially of, nucleic acids. This enables efficiently generating the backbone with the aptamers. Preferably, the aptamer is a nucleic acid-based aptamer. This enables efficiently generating a range of aptamers that are each specific to a particular labelling moiety. Aptamers may be generated in various ways, with SELEX (Systematic Evolution of Ligands by EXponential Enrichment) being the most typical route. Additionally, the backbone and/or the aptamer may comprise modifications, such as glycosylation. Further, it may be particularly preferred that the backbone with the aptamer comprises a naturally occurring D-DNA or a nucleic acid analogue such as non-naturally occurring nucleic acid, LNA, L-DNA, PT-DNA, or PNA or any other xeno nucleic acid (XNA), which may comprise modifications of the backbone and/or nucleobases.

In particular, the use of aptamers enables a compact label due to the generally small size of aptamers, which is in a range between 20 to 100 nucleotides, or between 10 to 50 kDa. Further, the ability to rapidly produce nucleic acid-based aptamers that have high affinity and/or specificity to a particular labelling moiety enable generating compact labels that may be efficiently generated.

Preferably, the backbone comprises at least two aptamers. Each aptamer may bind a labelling moiety. This enables generating labels with different or the same detectable properties and therefore generating a plurality of distinguishable labels or labels being easier to detect than a label with only one single labelling moiety.

Preferably, the aptamer of the backbone is a multimer. The multimer is preferably the same nucleic acid molecule. Alternatively, the multimer may comprise several individual monomers each monomer configured to either bind a labelling moiety with identical detectable properties or configured to bind labelling moieties with different detectable properties. This enables generating combinatorial labels or labels being easier to detect than a label with only one single labelling moiety. Preferably, each monomer binds to a single labelling moiety.

Preferably, the label comprises at least two labelling moieties. In particular, each labelling moiety is bound to one of the aptamers. The labelling moieties may have different or the same detectable properties, for example optical properties such as excitation/emission wavelength, or fluorescent lifetime. This enables generating combinatorial labels or labels being easier to detect than a label with only one single labelling moiety.

Preferably, the labelling moiety is a fluorescent protein or a molecular fluorophore. In particular, the molecular fluorophore may have a molecular weight in a range between 100 to 1000 Da. This enables a compact label. The fluorescent protein may have a molecular weight in a range between 10 and 100 kDa, for example. Fluorescent labelling moieties are optically detectable, for example by means of a fluorescent microscope, a plate reader, or cytometer, and may be distinguished by their optical properties.

Preferably, the labelling moiety comprises a biotin, for example the labelling moiety may be a biotinylated molecular fluorophore, and the at least one aptamer is configured to specifically bind the biotin, in particular, the at least one aptamer may bind the biotin of the labelling moiety by the complex structure of the at least one aptamer. This enables efficient assembly of the label from its parts, in particular from the at least one aptamer and the labelling moiety.

Preferably, the labelling moiety is a SMILE or dye-macrocycle cocrystal, a polymer dot or quantum dot. In this case it is advantageous to use an aptamer to recognize such particles directly, as this may be easier than functionalizing such particles.

Alternatively or in addition, the labelling moiety may comprise a polyyne detectable by Raman spectroscopy. The labelling moiety may also comprise a particle detectable by Raman spectroscopy, for example particles that are used for surface enhanced Raman spectroscopy (SERS). Such particles typically contain a gold (Au) or silver (Ag) core, a Raman reporter molecule, a shell made of for example polyethylene glycol (PEG). SERS probes are of great interest in microscopy, because of their very narrow emission peak, which may allow imaging for example +100 SERS probes in one multiplexed biomarker imaging experiment. Likewise, these probes are of great interest in the context of liquid proteomics and multiplexed immunoassays. In such assays the antibodies may be conjugated via for example NHS (N-hydroxysuccinimide) chemistry to the PEG, which is then used to coat the metallic nanoparticles. Using the strategy presented in this document allows for easy, rapid, and cost-effective conjugation of a number of antibodies or other affinity reagents with SERS particles.

Preferably, the backbone comprises an attachment oligonucleotide configured to attach to an affinity reagent of a marker, in particular, the attachment oligonucleotide may be configured to hybridise to a complementary oligonucleotide of an affinity reagent of a marker. This enables efficiently generating a marker from the label.

Preferably, the backbone comprises a cleavage site. This enables inactivating the label, for example, during iterative or cyclical labelling methods. The cleavage site may be configured to be cleavable by a cleavage agent, in particular. For example, the cleavage site may be a specific restriction site or a chemically/photochemically cleavable moiety.

Preferably, the cleavage site is arranged and configured to separate the attachment oligonucleotide of the label and the at least one aptamer of the label from each other upon cleavage of the cleavage site. Preferably, the cleavage of the cleavage site is irreversible. This enables efficiently separating the label from an entity such as an affinity reagent attached to the attachment oligonucleotide.

In another aspect, a marker is provided for analysing a biological sample. The marker comprises at least one label, in particular according to one of the embodiments described above, and an affinity reagent configured to bind specifically to a target analyte of the biological sample. In particular, the label is attached to the affinity reagent. This enables providing a marker with a compact label.

Preferably, the affinity reagent is an aptamer. This enables a providing a compact marker. The aptamer is in particular configured to bind to the respective target analyte of the biological sample. Alternatively, the affinity reagent could be an antibody, a single-domain antibody (also known as nanobody), a combination of at least two single-domain antibodies, an oligonucleotide, a morpholino, a PNA complementary to a predetermined RNA or DNA target sequence, a ligand, or a polymeric binder (e.g. a drug or a drug-like molecule), or a toxin, e.g. Phalloidin a toxin that binds to an actin filament.

In particular, the aptamer is configured to bind specifically to the target analyte due to the complex structure of the aptamer and based on intermolecular forces between the aptamer and the target analyte. Specifically, individual, discontinuous nucleotides of the nucleic acid of the aptamer bind to the target analyte, rather than continuous nucleotides of a sequence of the nucleic acid hybridising to the target analyte.

Preferably, the label is attached to the affinity reagent covalently. This enables a robust and compact marker.

Preferably, the affinity reagent comprises a complementary attachment oligonucleotide, which is complementary to the attachment oligonucleotide of the label. This enables efficient attachment of the label to the affinity reagent by hybridisation.

In a further aspect, a reagent kit for analysing a biological sample is provided. The reagent kit comprising at least one label, in particular according to an embodiment described above, and a cleavage agent configured to cleave a cleavage site of the label.

Preferably, the reagent kit comprises an affinity reagent configured to bind specifically to a target analyte of the biological sample. This enables efficiently generating a marker with the reagent kit.

In another aspect, a backbone comprising at least one aptamer is provided configured to specifically bind to at least one labelling moiety by the aptamer's complex structure, in order to generate a label, in particular as disclosed above. Preferably, the backbone comprises at least two aptamers, each configured to specifically bind a labelling moiety.

In a further aspect, a label kit for analysing a biological sample is provided comprising the backbone, in particular as described above, and the at least one labelling moiety.

### Terms and definitions

**"Dye":** In the sense of this document the terms "fluorescent dye", "fluorophore", "fluorochrome", "dye" are used interchangeably to denote a fluorescent chemical compound or structure and can be in particular one of the following: a fluorescent organic dye, a fluorescent quantum dot, a fluorescent dyad, a fluorescent carbon dot, graphene quantum dot or other carbon-based fluorescent nanostructure, a fluorescent protein, a fluorescent DNA origami-based nanostructure. From the organic fluorescent dyes in particular derivatives of the following are meant by the term "fluorescent dye": xanthene (e.g. fluorescein, rhodamine, Oregon green, Texas), cyanine (e.g. cyanine, indocarbocyanine, oxacarbocyanine, thiacarbocyanine, merocyanine), derivatives, squaraine rotaxane derivatives, naphthalene, coumarin, oxadiazole, anthracene (anthraquinones, DRAQ5, DRAQ7, CyTRAK Orange), pyrene (cascade blue), oxazine (Nile red, Nile blue, cresyl violet, oxazine 170), acridine (proflavine, acridine orange, acridine yellow), arylmethine (auramine, crystal violet, malachite green), tetrapyrrole (porphin, phthalocyanine, bilirubin), dipyrromethene (BODIPY, aza-BODIPY), a phosphorescent dye, or a luminescent dye. The following trademark groups designated commercially available fluorescent dyes, which may include dyes belonging to different chemical families CF dye (Biotium), DRAQ and CyTRAK probes (BioStatus), BODIPY (Invitrogen), EverFluor (Setareh Biotech), Alexa Fluor (Invitrogen), Bella Fluore (Setareh Biotech), DyLight Fluor (Thermo Scientific), Atto and Tracy (Sigma-Aldrich), FluoProbes (Interchim), Abberior Dyes (Abberior Dyes), Dy and MegaStokes Dyes (Dyomics), Sulfo Cy dyes (Cyandye), HiLyte Fluor (AnaSpec), Seta, SeTau and Square Dyes (SETA BioMedicals), Quasar and Cal Fluor dyes (Biosearch Technologies), SureLight Dyes (Columbia Biosciences), Vio Dyes (Milteny Biotec) [list modified from: https://en.wikipedia.org/wiki/Fluorophore]. From the group of fluorescent proteins in particular the members of the green fluorescent protein (GFP) family including GFP and GFP-like proteins (e.g DsRed, TagRFP) and their (monomerized) derivatives (e.g., EBFP, ECFP, EYFP, Cerulaen, mTurquoise2, YFP, EYFP, mCitrine, Venus, YPet, SuperfolderGFP, mCherry, mPlum) are meant by the term "fluorescent dye" in the sense of this document. Further from the group of fluorescent proteins the term "fluorescent dye" in the sense of this document may include fluorescent proteins, whose absorbance or emission characteristics change upon binding of ligand like for example BFPms1 or in response to changes in the environment like for example redox-sensitive roGFP or pH-sensitive variants. Further from the group of fluorescent proteins the term "fluorescent dye" in the sense of this document may include derivative of cyanobacterial phycobiliprotein small ultra red fluorescent protein smURFP as well as fluorescent protein nanoparticles that can be derived from smURFP. An overview of fluorescent proteins can be found in Rodriguez et al. 2017 in Trends Biochem Sci. 2017 Feb; 42(2): 111-129. The term "fluorescent dye" in the sense of this document may further refer to a fluorescent quantum dot. The term "fluorescent dye" in the sense of this document may further refer to fluorescent carbon dot, a fluorescent graphene quantum dot, a fluorescent carbon-based nanostructure as described in Yan et al. 2019 in Microchimica Acta (2019) 186: 583 and Iravani and Varma 2020 in Environ Chem Lett. 2020 Mar 10 : 1-25. The term "fluorescent dye" in the sense of this document may further refer to a fluorescent polymer dot (Pdot) or nanodiamond. The term "fluorescent dye" in the sense of this document may further refer to a fluorescent dyad, like for example a dyad of a perylene antenna and a triangelium emitter as described in Kacenauskaite et al. 2021 J. Am. Chem. Soc. 2021, 143, 1377-1385.

The term "fluorescent dye" in the sense of this document may further refer to an organic dye, a dyad, a quantum dot, a polymer dot, a graphene dot, a carbon-based nanostructure, a DNA origami-based nanostructure, a nanoruler, a polymer bead with incorporated dyes, a fluorescent protein, an inorganic fluorescent dye, a SMILE, or a microcapsule filled with any of the aforementioned.

The term "fluorescent dye" in the sense of this document may further refer to a FRET-pair having at least one fluorescent dye as FRET donor and at least one fluorescent dyes as a FRET acceptor, or a FRET-triple, which is used to generate a three component Förster resonance energy transfer. In particular, the FRET-pair or FRET-triplet is connected by a complementary linker or by a linking element.

The term "fluorescent dye" in the sense of this document may further refer to a FRET n-tupel of physically connected dyes.

**"Predetermined target structure":** In the sense of this document "predetermined target structure" refers to a target molecule or a target structure or to an analyte, which may for example be a protein (e.g. a certain protein), an RNA sequence (e.g. the mRNA of a certain gene), a peptide (e.g. somatostatin), a DNA sequence (e.g. the a genetic locus or element), a metabolite (e.g. lactic acid), a hormone (e.g. estradiol), a neurotransmitter (e.g. dopamine), a vitamin (e.g. cobalamine), a micronutrient (e.g. biotin), a metal ion (e.g. metal and heavy metal ions like Cd(II), Co(II), Pb(II), Hg(II), U(VI)).

**"Sample":** In the sense of this document "sample" refers to a biological sample which may also be named a biological specimen including, for example blood, serum, plasma, tissue, bodily fluids (e.g. lymph, saliva, semen, interstitial fluid, cerebrospinal fluid), feces, solid biopsy, liquid biopsy, explants, whole embryos (e.g. zebrafish, Drosophila), entire model organisms (e.g. zebrafish larvae, Drosophila embryos, C. elegans), cells (e.g. prokaryotes, eukaryotes, archea), multicellular organisms (e.g. Volvox), suspension cell cultures, monolayer cell cultures, 3D cell cultures (e.g. spheroids, tumoroids, organoids derived from various organs such as intestine, brain, heart, liver, etc.), a lysate of any of the aforementioned, a virus. In the sense of this document "sample" further refers to a volume surrounding a biological sample. Like for example in assays, where secreted proteins like growth factors, extracellular matrix constituents are being studied the extracellular environment surrounding a cell up to a certain assay-dependent distance, is also referred to as the "sample". Specifically, affinity reagents brought into this surrounding volume are referred to in the sense of this document as being "introduced into the sample".

### Short Description of the Figures

Hereinafter, specific embodiments are described referring to the drawing, wherein:

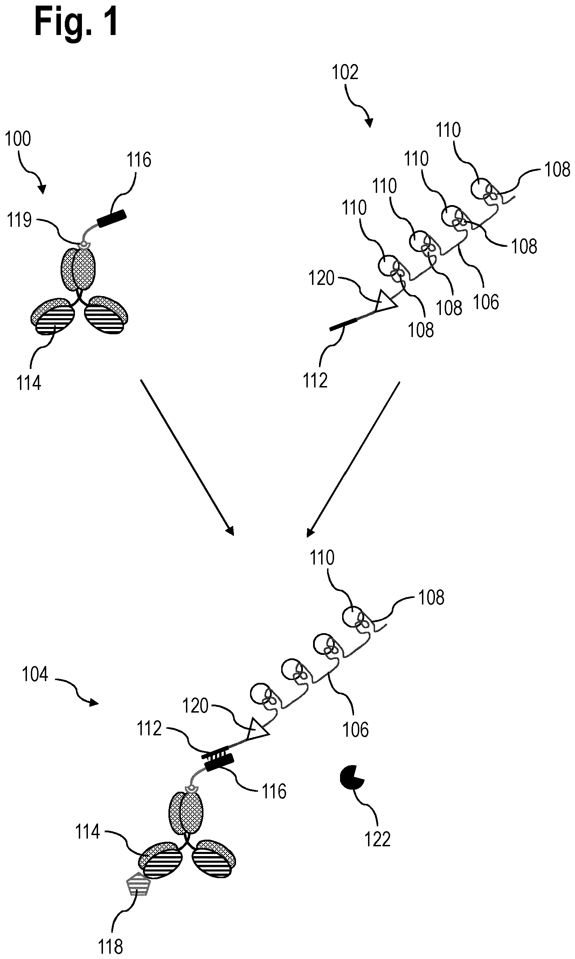

- Figure 1: is a schematic view an affinity reagent, a label and a marker comprising the affinity reagent and the label.

### Detailed Description

Figure 1 is a schematic view an affinity reagent 100, a label 102 and a marker 104 comprising the affinity reagent 100 and the label 102.

The label 102 comprises a backbone 106. The backbone 106 comprises aptamers 108, in particular four aptamers 108, each aptamer 108 configured to specifically bind to a labelling moiety 110. The backbone 106 preferably comprises nucleic acids and the aptamers 108 are nucleic acid aptamers 108.

Alternatively, the backbone 106 may be the aptamer 108, in particular if the label 102 only comprises a single aptamer 108. In a further alternative or additionally, the aptamers 108 may be connected to each other via a nucleic acid of the backbone 106.

Thus, the aptamers 108 may either be formed from a single continuous nucleic acid strand or each aptamer 108 may be formed from a separate single nucleic acid strand and the aptamers 108 are connected to each other via a nucleic acid of the backbone 106, which hybridises to two adjacent aptamers 108, for example.

The labelling moieties 110 are identical, in particular with respect to their detectable properties. For example, the labelling moieties 110 may be optically detectable fluorophores that have identical fluorescent excitation and emission wavelengths, as well as fluorescent lifetime.

Alternatively, the label 102 may comprise several aptamers 108 that are configured to specifically bind to different labelling moieties 110. For example, the label 102 may alternatively comprise two aptamers 108 that are each configured to bind specifically to either a first or a second labelling moiety 110 and the first and the second labelling moiety differ from each other in at least one detectable property. In case the first and the second labelling moieties are fluorophores, they may differ in at least one of a fluorescent excitation and emission wavelengths, or a fluorescent lifetime.

The labelling moieties 110 may be a fluorophore, for example, a fluorescent protein and/or a molecular fluorescent. A molecular fluorophore, such as a carbon-based fluorescent nanostructure, a xanthene derivative, or a quantum dot, generally has a molecular weight in a range between 100 to 1000 Da. In contrast a fluorescent protein, such as green fluorescent protein, generally has a molecular weight in a range between 10 kDa and 100 kDa.

The labelling moieties 110 may also be fluorogenic compounds, which are configured to become fluorescent through a chemical reaction with a specific target molecule.

Alternatively or additionally, the labelling moieties 110 may comprise polyynes detectable by Raman spectroscopy. This enables generating labels that may be uniquely identified by Raman spectroscopy.

The aptamers 108 may form or fold into a complex structure or conformation. In particular, the complex structure of the aptamers 108 may be a secondary, tertiary and/or quaternary structure of the backbone 106. Thus, the aptamers 108 each have a secondary, tertiary or quaternary structure, in particular. The aptamers 108 bind specifically to the respective labelling moiety 110 due to the complex structure of the aptamer 108. Specifically, individual, discontinuous nucleotides of the nucleic acid of the aptamers 108 bind to the respective labelling moiety 110. This binding may comprise intermolecular forces such as hydrogen bonding, dipole-dipole interactions, and van der Waals forces. This is in contrast to a nucleic acid hybridising, which is characterised by a continuous sequence of nucleotides of the nucleic acid hybridising to a particular target, in particular a complementary continuous nucleotide sequence.

The binding of the aptamers 108 to the respective labelling moiety 110 based on the complex structure enables particular high affinity binding and specificity to the respective labelling moiety 110.

The affinity reagent 100 is an antibody 114. The antibody is configured to specifically bind to a target analyte 118 in a biological sample (not shown). Alternatively, the antibody 114 may be an aptamer or an antibody fragment.

The affinity reagent 100 further comprises an oligonucleotide 116. The oligonucleotide 116 is complementary to an attachment oligonucleotide 112 of the label 102. The attachment of the label 102 to the affinity reagent 100 via the attachment oligonucleotide 112 and the complementary oligonucleotide 116 generates the marker 104, see the bottom part of Fig. 1. This enables easy and specific attachment of the label 102 to the affinity reagent 100 by hybridisation. This is of particular benefit when generating a library of large numbers of different markers 104. The particular sequence of the attachment oligonucleotide 112 may be specific to the identity of the labelling moieties 110 and/or the antibody 114.

The marker 104 enables specifically binding the label 102 to a target analyte 118, in particular a protein, of a biological sample via the affinity reagent 100, for example, in order to detect, identify, and/or localise the target analyte 118 in the biological sample.

Alternatively, the label 102, in particular, the backbone 106, may be attached to the affinity reagent 100 covalently (not shown).

In a further alternative, the label 102 may be directly attached to a nucleic acid target analyte of the biological sample via the attachment oligonucleotide 112 based on hybridisation of the attachment oligonucleotide 112 to a complementary sequence of the nucleic acid target analyte. In this case, the attachment oligonucleotide 112 has the functionality of an affinity reagent.

The complementary oligonucleotide 116 of the affinity reagent 100 may optionally be attached to the antibody 114 of the affinity reagent 100 by a near covalent interaction between a streptavidin-biotin pair 119. To that end, the complementary oligonucleotide 116 may be covalently attached to one of (monovalent) streptavidin and biotin and the antibody 114 of the affinity reagent 100 may be covalently attached to the other one of (monovalent) streptavidin and biotin. When assembling the affinity reagent 100, the streptavidin binds to the biotin resulting in the attachment of the complementary oligonucleotide 116 to the antibody 114. This may be particularly advantageous, when a large library of affinity reagents 100 and markers 104 are assembled.

Optionally, the backbone 106 of the label 102 may further comprise a cleavage site 120. The cleavage site 120 is configured to be specifically cleavable by a cleavage agent 122. The cleavage site 120 is preferably arranged between the attachment oligonucleotide 112 and the backbone 106, in particular the aptamers 108, of the label 102. This enables separating, preferably irreversibly, the aptamers 108 with the labelling moieties 110 from the attachment oligonucleotide 112 and therefore from the affinity reagent 100. This may be particularly advantageous when using the label 102 or the marker 104 for iterative staining methods for biological samples.

The cleavage site 120 may be a particular sequence of the backbone 106 that is specifically cleaved by a restriction enzyme 122, for example. Alternatively, the cleavage 120 site may be a photocleavable moiety cleavable by illuminating with light of a particular wavelength and/or intensity, for example.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100: Affinity reagent
- 102: Label
- 104: Marker
- 106: Backbone of label
- 108: Aptamer of label
- 110: Labelling moiety
- 112: Attachment oligonucleotide
- 114: Antibody of affinity reagent
- 116: Complementary oligonucleotide
- 118: Target analyte
- 119: Streptavidin-biotin pair
- 120: Cleavage site
- 122: Cleavage agent

## Claims

1. A label (102) for analysing a biological sample comprising:
a backbone (106) comprising at least one aptamer (108), and
at least one labelling moiety (110),
wherein the aptamer (108) is configured to specifically bind to at least one labelling moiety (110) by its complex structure.

2. The label according to claim 1, wherein the backbone (106) comprises nucleic acids.

3. The label according to one of the preceding claims, wherein the backbone (106) comprises at least two aptamers (108).

4. The label according to one of the preceding claims, wherein the aptamer (108) of the backbone (106) is a multimer.

5. The label according to one of the preceding claims, comprising at least two labelling moieties (110).

6. The label according to one of the preceding claims, wherein the labelling moiety (110) is a fluorescent protein or a molecular fluorophore.

7. The label according to one of the preceding claims, wherein the labelling moiety (110) comprises a biotin and the at least one aptamer (108) is configured to specifically bind the biotin.

8. The label according to one of the preceding claims, wherein the backbone (106) comprises an attachment oligonucleotide (112) configured to attach to an affinity reagent (100) of a marker (104).

9. The label according to one of the preceding claims, wherein the backbone (106) comprises a cleavage site (120).

10. The label according to claim 9, wherein the cleavage site (120) is arranged and configured to separate the attachment oligonucleotide (112) and the at least one aptamer (108).

11. A backbone (106) comprising at least one aptamer (108) configured to specifically bind to at least one labelling moiety (110) by the aptamer's complex structure, in order to generate a label (102) according to one of the preceding claims.

12. A marker (104) for analysing a biological sample comprising:
at least one label (102) according to one of the preceding claims, and
an affinity reagent (100) configured to bind specifically to a target analyte (118) of the biological sample.

13. The marker according to claim 12, wherein the affinity reagent (100) is an aptamer.

14. The marker according to one of the preceding claims 12 or 13, wherein the label (102) is attached to the affinity reagent (100) covalently.

15. The marker according to one of the preceding claims 12 or 13, wherein the affinity reagent (100) comprises a complementary oligonucleotide (116), which is complementary to the attachment oligonucleotide (112) of the label (102).

16. A reagent kit for analysing a biological sample comprising:
at least one label (102) according to claims 9 or 10, and
a cleavage agent (122) configured to cleave the cleavage site (120) of the label (102).

17. The reagent kit according to claim 16, comprising an affinity reagent (100) configured to bind specifically to a target analyte (118) of the biological sample.

18. A label kit, for analysing a biological sample comprising:
the backbone (106) of claim 11, and
the at least one labelling moiety (110).
